# EUROPEAN PATENT APPLICATION

(11) **EP 3 159 016 A1**
(43) Date of publication of application: **26.04.2017**
(21) Application number: 16194739.5
(22) Date of filing: 20.10.2016
(51) Int. Cl.: A61L 24/06, A61L 24/00

(54) **NEOPRENE MEDICAL DEVICE**

(30) Priority: 20.10.2015 IT UB20154805
(71) Applicant: Mazzaferro, Vincenzo Maria, 20134 Milano (IT)
(72) Inventor: Mazzaferro, Vincenzo Maria, 20134 Milano (IT)
(74) Representative: Calogero, Ida

(57) **Abstract**

The present invention relates to a neoprene-based medical device for use in the medical field, particularly as occlusive material to induce chemical pancreatectomy of the exocrine component of pancreas during surgeries that involve the removal of the head of the pancreas, duodenum or terminal bile duct.

## Description

The present invention relates to a neoprene-based medical device for use in the medical field, particularly as occlusive material to induce chemical pancreatectomy of the exocrine component of pancreas during surgeries that involve the removal of the head of the pancreas, duodenum or terminal bile duct. Polychloroprene [poly(2-chloro-1.3-butadiene)], more commonly known as neoprene (trade name of the company DuPont Performance Elastomers for a family of synthetic rubbers based on the polymer form of chloroprene) is one of the leading synthetic elastomers with a worldwide annual consumption of about 300,000 tons.

The main features are elasticity, resistance to cutting and crushing, resistance to weathering and heat, and it also is inert to many chemicals, oils and solvents.

For these reasons, it has important applications in different technology areas, mainly in the synthetic rubber industry, but also as a raw material for adhesives (about 33%) or for medical applications as an alternative polymer to LaTeX in the production of gloves (about 6%).

The surgical approach codified worldwide for treatment of primary tumors of the head of the pancreas, duodenum, the papilla and the distal bile duct consists in the surgery called duodenocefalopancreasectomy (DCP or Whipple procedure), i.e. the demolition of a single block of the duodenum, the head of the pancreas, the extrahepatic bile duct, the distal third of the stomach and first jejunal loop. Although refined in many respects, DCP surgery is burdened by significant morbidity, related especially to the delicate phase following the removal of the tumor, consisting of a series of reconstructions of the biliary, gastro-enteric and above all pancreatic continuity. Among the many variants of the reconstructive phase, that of reconnection (anastomosis) between the body and tail of the pancreas and the intestines (pancreatic-jejunum anastomosis) turns out to be the most at risk and with a higher rate of complications.

Among these, the most frequent (20-40% of patients undergoing DCP) are pancreatic-jejunal anastomotic dehiscence, the onset of pancreatic fistulas and stump pancreatitis, all due to the loss of pancreatic juice, notoriously very caustic, from the reconstructions sites. It should be noted that the management of this type of complication is sometimes very complex and burdened by the need of re-surgery of various importance, still at significant risk of removal of the entire pancreas, associated with long hospital stays in patients already very compromised who unfortunately can also die due to fatal complications.

It is therefore desirable, in order to reduce the perioperative morbidity and mortality related to pancreatic fistula formation, especially in patients at risk (e.g. the elderly, with significant comorbidity or unfavorable anatomical or cancer conditions), to implement alternatives procedures to conventional pancreatic-jejunal anastomosis. All this in order to both reduce the complexity of conventional surgery in patients at high risk of not being able to withstand it and possibly shorten the postsurgical recovery time in patients with co-morbidity or very unfavorable prognosis for cancer, i.e. for tumors with high probability of relapse in a short time.

Alternative intervention approaches have been developed in the past, aimed to occlude the pancreatic duct with adhesive, such as using Ethibloc®, an alcohol prolamin (Gebhardt C. et al., Horm. Metab. Res. Suppl. 1983:9-11) or with the fibrin-based adhesive solution Tissucol® (Idezuki Y et al. Am. J. Surg. 1969; 117: 33-39). However, it is known that using these preparations variously employed, it has not been possible to significantly reduce the formation of pancreatic fistulae (see Suc B. et al, Ann. Surg. 2003; 237: 57-65).

In preliminary experiments conducted by Di Carlo et al. in 1989 on 50 patients, the pancreatic duct occlusion with neoprene adhesive was tested after Whipple procedure, highlighting pancreatic atrophy and complete loss of exocrine function of the residual pancreatic gland among the results (Di Carlo V. et al. World J. Surg. 1989, Vol. 13:105-111). More recently, this type of surgery with neoprene in the reconstructive phase after pancreatectomy has been discouraged because of the many complications and high morbidity recorded (Benoni E. et al. J. Gastrointestin. Liver Dis., Marzo 2008, Vol. 17: 43-47).

In order to obviate the problems arising after the surgery of demolition of the head of the pancreas, to simplify the execution thereof, reduce the clinical and management weight of these operations and ensure a better quality of life for the short and medium term in patients at risk of long postoperative hospital stay, the author of the present invention has developed a formulation of occlusive material of the residual pancreatic duct based on poly(2-chloro-1,3-butadiene) (neoprene) to be applied after surgical removal of the cephalic portion of the pancreas or in the treatment of pancreatic fistula requiring surgical revision, which does not show the limitations emerged from the known surgical techniques.

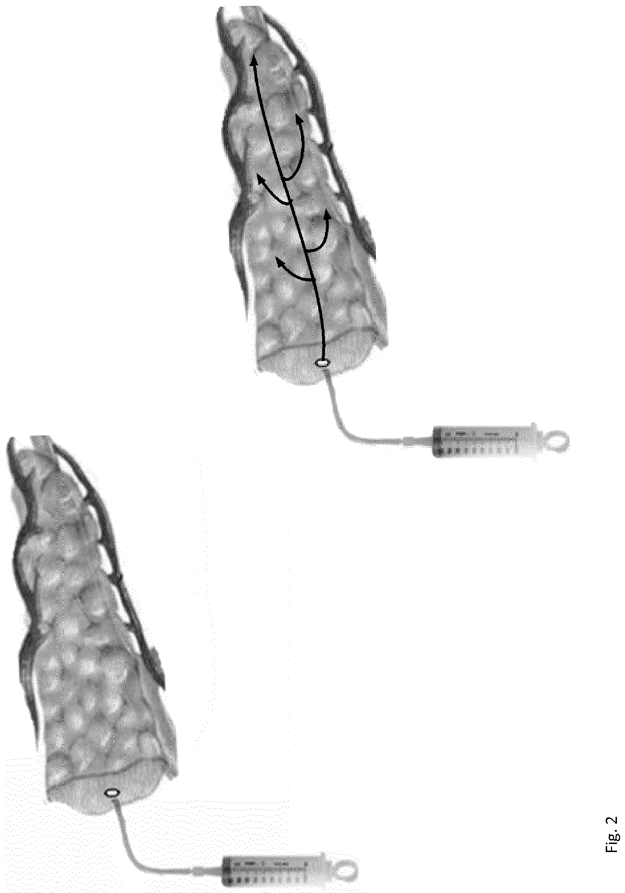

Once stabilized, the neoprene-based formulation is injected in liquid form, in a sterile manner and manually by the surgeon in the residual pancreatic duct (with a system consisting of sterile syringe and connectors as shown in figure 2) and within a few minutes it polymerizes, changing to the semi-solid state. In practice, the neoprene injected reaches, up to the most peripheral terminations, the system - anatomically conceivable as a tree of collection channels - that conveys the pancreatic juice produced by the pancreatic cells up to the main manifold (called Wirsung duct) which flows into the intestine at the level of the portion thereof called the duodenum. The manual and delicate inoculation of limited amounts of neoprene (7 to 15 cc) in the residual pancreatic duct in fact blocks the discharge of pancreatic juice from the Wirsung duct, which can then no longer be reconnected to the intestine, avoiding all the serious complications listed above as frequent cause of postoperative morbidity and mortality.

The injection of the device in the pancreatic duct of the residual gland stump therefore induces a chemical pancreatectomy of the exocrine component of the pancreas. This effect is actually the condition that the surgeon can judge as the most desirable and necessary in order to reduce the chance of complex postoperative fistulae in patients at risk of complications or difficult from a technical point of view (that is, reducing the losses of pancreatic juice from the residual gland stump post-surgery). This attitude favored by the intended use of neoprene has important clinical implications, such as the post-surgical recovery time reduction and the optimization of intra- and extra-hospital management in patients at high risk of complications and not otherwise treatable.

In the patients treated by the author of the present invention, the intraductal injection of the device after pancreaticoduodenectomy has proven successful and competitive compared to the conventional approach represented by the pancreatic-jejunal anastomosis.

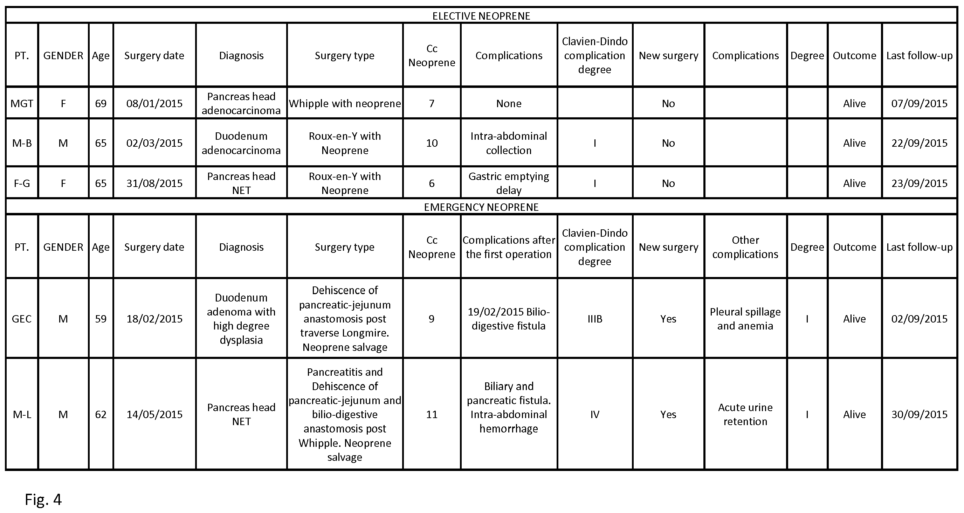

Since we started the testing approved by the Ethics and Scientific Committee of the National Cancer Institute of Milan of the neoprene inoculation method in Wirsung duct, 5 patients have been treated (3 elective and 2 in emergency) whose outcome is summarized in the table shown in Figure 4. Using the neoprene-based medical device according to the present invention, excellent results have been obtained in terms of control of complications and duration of hospitalization up to the positive final outcome of survival obtained in all the treated cases.

No treated patient developed postsurgical diabetes and in no case local toxicity events were recorded at either the inoculation site or in other sites. Despite the weight of the procedure (and in 20% of them the urgent need to halt a serious complication), all patients were successfully treated and all are alive at home after discharge.

Therefore, the object of the present invention is a sterile aqueous dispersion comprising 55% of poly (2-chloro-1,3-butadiene) or neoprene characterized in that it is stabilized at pH comprised in a range between 13-13.5 by addition of potassium hydroxide, for use in medical field. The pH of the dispersion is highly critical: as soon as such a dispersion comes into contact with the pancreatic fluid rich in sodium bicarbonate, the polymer part of the dispersion (i.e. the neoprene) undergoes a gelling reaction within a few minutes, thereby passing from the liquid state to gel, with occlusion of the pancreatic juice discharge from the pancreas duct. The stability of the product pH is guaranteed for the entire validity of the same. Conversely, the pH of commercially available neoprene-based dispersions for intended use other than medical-surgical lowers drastically during storage. According to a preferred embodiment, the sterile aqueous solution has a volume of between 3 and 12 mL.

Preferably, the sterile aqueous solution according to the invention is packaged in a disposable sterile glass container in the absence of oxygen. Even more preferably, such a glass container is a glass vial. Nitrogen is introduced in the headspace of the vial to eliminate the oxygen, thereby increasing the stability of the product over time. In a preferred embodiment of the invention, the vial is an amber hydrolytic degree vial.

The invention is therefore characterized by:
a) a chemical adaptation of the original product in order to be used in the selected anatomical district (pancreas);
b) an intended use in the medical field that is completely different from the original product;
c) a precise use in terms of type of eligible diseases, general and oncological features of the patients and methods of use.

The above neoprene-based sterile aqueous dispersion is in fact in the medical field an occluding adhesive for the pancreatic duct in patients undergoing pancreaticoduodenectomy due to cancer or other serious pancreas, duodenum and terminal bile duct diseases. In particular, the above adhesive has its intended use in patients with high risk of complications, or unfavorable anatomical conditions or high risk for tumor recurrence in the short term or in the case of postoperative pancreatic fistula formation, or loss of pancreatic juice from the conventional surgical reconstruction that follows the phase of demolition of the anatomical region containing the tumor or is site of other pathology not otherwise treatable. From a technical point of view (method of use), the neoprene dispersion should be injected manually at low pressure via a 5 mL syringe attached to an intravenous catheter suitably advanced in the Wirsung duct of the residual pancreas (see Figure 2).

The catheter should be pushed as distally as possible within the gland and hence be slowly retracted during the injection.

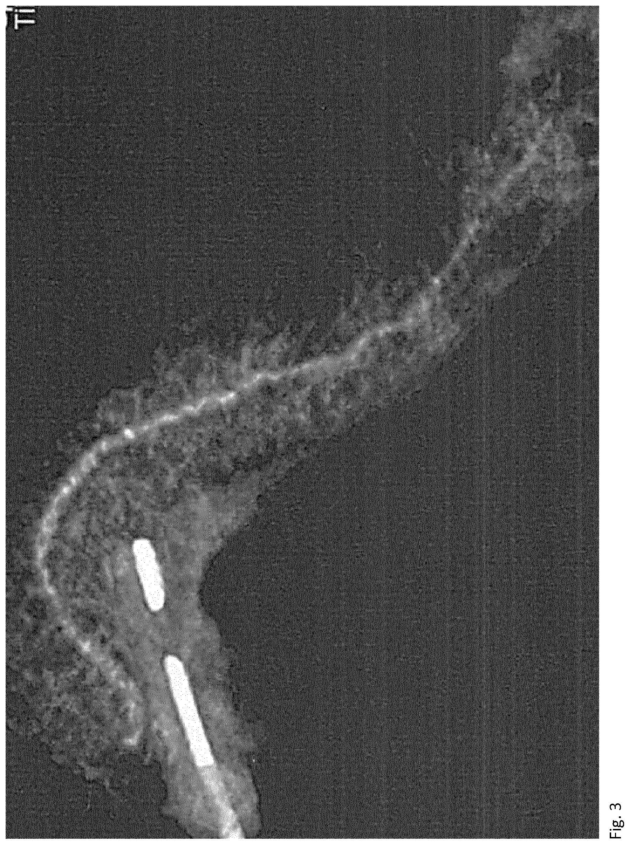

Since the product in contact with the pancreatic juice, contained in the Wirsung and its division branches, slowly tends to polymerize, the surgeon should check the transformation of the dispersion from the liquid state to semi-solid glue, able to create a mold of the intrapancreatic duct division tree (see Figure 3).

The amount of device to be injected is variable, depends on the size of the pancreas and the gauge of the duct of every single case and can range from 3 mL to 12 mL for each patient.

Inoculations of less than 3 mL in an adult patient should be considered failures of the procedure and breaches of the chemical pancreatectomy protocol.

The object of the present invention therefore is a medical device for occluding the pancreatic duct in patients undergoing pancreaticoduodenectomy, comprising the neoprene-based sterile aqueous dispersion described above. This device is sterile and disposable. Preferably, said medical device comprises the sterile aqueous solution packaged in glass vials containing nitrogen in the headspace of the vial. Moreover, the present invention also refers to a complete kit for the occlusion of the pancreatic duct in patients undergoing pancreaticoduodenectomy (or surgery on the region of the head of the pancreas, duodenum and distal bile duct) comprising the following components:
i) the medical device comprising the neoprene-based sterile aqueous dispersion described above;
ii) an intravenous catheter;
iii) a syringe.

According to a preferred embodiment of the present invention, the intravenous catheter ii) is highly flexible, preferably of silicone with a gauge of between 14-18 Fr (e.g. nutricath 1.3-2.0 mm, Vygon®, Ecouen, France) and a length of between 25-35 cm. Even more preferably, said intravenous catheter is connected to syringe iii) via a calibrated connector, preferably a three way connector. The volume of the neoprene dispersion injected by the syringe is between 3-12 mL, preferably 5 mL.

The kit according to the invention can further comprise locking systems of the inoculum site (e.g. tobacco bag wire type 5/0 prolene Ethicon® Johnson and Johnson) or dedicated clip or mini sponge to absorb any product residues leaked during the injection in order to increase the safety margins of the inoculum itself.

Finally, the invention contemplates a process for the preparation of the neoprene-based sterile aqueous dispersion described above, comprising the following steps:
a) finding or preparation of an aqueous mixture of 55% poly(2-chloro-1,3-butadiene) (such as Dispercoll C84, Bayer) and mechanical stirring of the same;
b) bubbling with filtered nitrogen (i.e. medical-grade nitrogen) to remove the oxygen;
c) pH stabilization in a range comprised between 13-13.5 by addition of potassium hydroxide;
d) insertion of the mixture into vials through addition of nitrogen into the headspace of the vial to remove the oxygen;
e) sterilization.

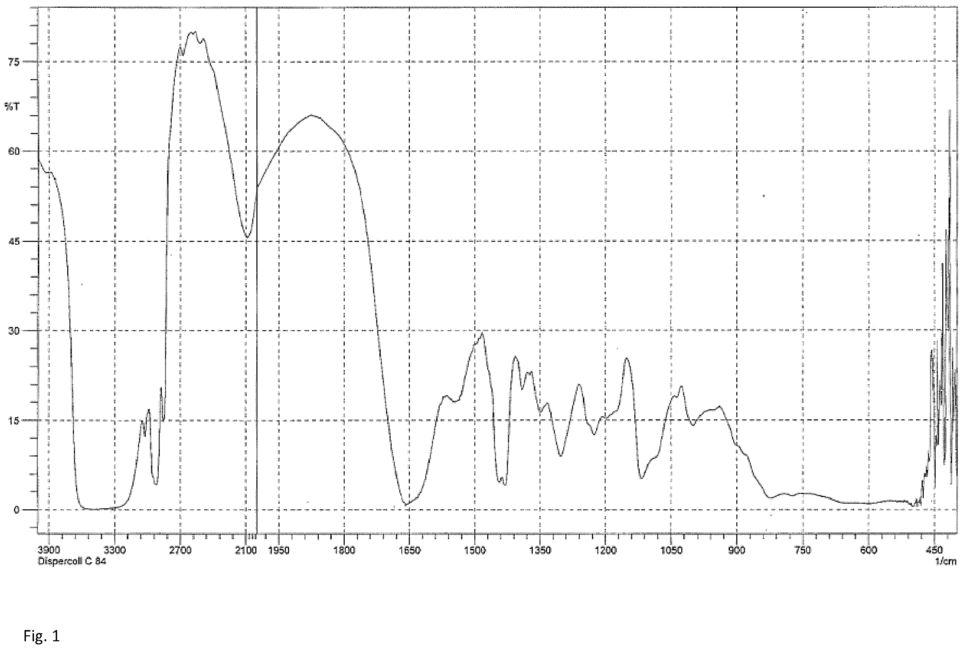

Preferably, the starting mixture at 55% neoprene of step a) is characterized by the IR spectrum shown in Figure 1. Even more preferably, the mixture Dispercoll C84 marketed by Bayer for industrial uses is used. Preferably, the raw material is subjected to mechanical stirring with a mechanical stirrer prior to the stabilization step.

The addition of nitrogen in step b) in cooperation with the stabilization with concentrated potassium hydroxide in step c) makes the product stable at room temperature and therefore autoclavable. Therefore, the stabilization of the molecule takes place with the combined action of KOH (and not for example NaOH) and filtered nitrogen.

It is noted that the same exemplary mixture (Dispercoll C84) commercially available for uses other than the object of the present invention is very unstable for the following reasons:
- pH: the pH as reported in the art released by the manufacturer tends to lower, thereby losing its hydrolytic attack function of the pancreatic juice;
- Oxygen: the mixture is sensitive to atmospheric oxygen, in fact if the product comes into contact with oxygen, the polymer fraction is separated from water with formation of clots;
- Temperature: the product is sensitive to temperatures of >25 °C and under thermal stress, the polymer fraction is separated from the aqueous fraction and therefore is not effective for pharmaceutical use. It is noted that the medical use to which the product proposed herein is intended is by definition used at different temperature, i.e. in the range of the body temperature of patients undergoing surgery, i.e. ≥ 36 °C.
- Microbiological contamination: the starting mixture in standard condition is not microbiologically pure and also cannot undergo sterilization processes to make it sterile.

Moreover, the product is sensitive to light, in fact in the presence of direct sunlight, the polymer fraction will degrade and separate from the aqueous fraction. Finally, in the presence of direct sunlight the product will degrade thereby losing its adhesive properties.

Among the steps of this process, the most critical one is step c) of pH stabilization of the dispersion between 13-13.5, which guarantees the gelling reaction when the polymer, once injected into the pancreatic duct, comes into contact with the pancreatic juice.

During the insertion step d), nitrogen is inserted into the headspace of the vial, which pushes the oxygen out, thus increasing the stability of the product over time. Nitrogen is in fact essential to reduce the oxygen levels that would cause the polymer phase separation from the aqueous phase during the sterilization step. The insertion of the product into vials consists in dividing the polymer mixture inside a vial.

Preferably, a hydrolytic degree amber vial is used. The distribution of the mixture in amber vials, therefore not permeable to light, can ensure greater product stability over time and retention of the adhesion properties.

More specifically, the process of insertion into vials involves the following steps:
1) collecting the vial;
2) washing with sterile water for injectable preparations (W.F.I.);
3) drying;
4) sterilization in an oven;
5) filling of the vials with the neoprene suspension at stabilized pH;
6) inserting a nitrogen stream into the headspace of the vial and sealing the vial;
7) placing the vials on the cart.

The sterilization of step e) of the production process according to the invention preferably takes place in steam autoclave (even more preferably at 115 °C for 35 minutes). This allows ensuring the product sterility and thus the absence of pathogenic microorganisms that for a particularly weak subject could lead to irreparable damage. Finally, the packaging and quality control analysis steps follow for batch release.

Overall, therefore, the production process according to the invention allows transforming the starting aqueous 55% neoprene dispersion (for example, Dispercoll C84, Bayer) from simple polymer for industrial use with unstable pH upon storage into a medical device usable as pancreatic duct occlusive material. The present invention will now be described by way of a non-limiting example on the basis of the results shown in the accompanying figures, in which:
- Figure 1 shows the IR spectrum of the neoprene used in the medical device according to the invention;
- Figure 2 shows a diagram of the Wirsung duct cannulation procedure after pancreatectomy which removed the "head" of the pancreas;
- Figure 3 shows the image of a TAC of a patient treated with the medical device described deposited and solidified within the residual pancreas;
- Figure 4 shows a table showing the results of treatment with neoprene-based medical device on 5 patients who were treated with the preparation, after informed consent and approval of the trial by the Ethics and Scientific Committee of the National Cancer Institute of Milan.

## Claims

1. Sterile aqueous dispersion comprising 55% of poly (2-chloro-1,3-butadiene), **characterized in that** it is stabilized at pH comprised in a range between 13-13.5 by addition of potassium hydroxide, for use in medical field.

2. Sterile aqueous dispersion for use in medical field according to claim 1, having a volume comprised between 3 - 12 ml.

3. Sterile aqueous dispersion for use in medical field according to any one of the claims 1-2, which is inserted in a disposable sterile glass container in the absence of oxygen, preferably a vial.

4. Sterile aqueous dispersion for use in medical field according to any one of the claims 1-3, as occlusive material of pancreatic duct in patients undergoing pancreaticoduodenectomy.

5. Sterile aqueous dispersion for use in medical field according to claim 4, wherein said patients are at high risk of post-surgical pancreatic fistula or at high cancer risk.

6. Medical device for treating patients undergoing pancreaticoduodenectomy, comprising the sterile aqueous dispersion according to any one of the claims 1-3.

7. Medical device for treating patients undergoing pancreaticoduodenectomy according to claim 6, wherein said sterile aqueous dispersion is contained in a glass vial containing nitrogen in the headspace.

8. Kit for treating patients undergoing pancreaticoduodenectomy, comprising the following components:
i) a medical device according to any one of the claims 6-7;
ii) an intravenous catheter;
iii) a syringe with a connector.

9. Kit according to claim 8, wherein said intravenous catheter ii) is made of silicone and has a gauge comprised between 14-18 Fr and a length comprised between 25-35 cm.

10. Process for the preparation of the medical device according to any one of the claims 6-7, comprising the following steps:
a) finding or preparation of an aqueous mixture of 55% poly(2-chloro-1,3-butadiene) and mechanical stirring of the same;
b) bubbling the mixture with filtered nitrogen;
c) pH stabilization in a range comprised between 13-13.5 by addition of potassium hydroxide;
d) insertion of the mixture into vials through addition of nitrogen into the headspace of the vial to remove the oxygen;
e) sterilization.
